# EUROPEAN PATENT APPLICATION

(11) **EP 4 840 410 A2**
(43) Date of publication of application: **18.11.2026**
(21) Application number: 26198023.9
(22) Date of filing: 31.07.2023
(51) Int. Cl.: A61B 17/17

(54) **EQUIPMENT FOR THE APPLICATION OF A TROCHLEAR PROSTHESIS**

(62) Document number(s) of the earlier application(s) in accordance with Art. 76 EPC: 23772939.7 / 4 753 624
(71) Applicant: INTRAUMA SPA, 10090 Bruino (TO) (IT)
(72) Inventor: DEL MEDICO, Nilli, 10090 BRUINO (TO) (IT)
(74) Representative: Robba, Pierpaolo

(57) **Abstract**

The invention relates to an equipment for implanting a trochlear prosthesis (1) that comprises a guide assembly (9) provided with at least one guide elements (9") having a handgrip (19) and a cannulated wire guide (21) able to slide axially inside the handgrip (19) and equipped with an axial through-hole (21a) adapted to accommodate therein a guide wire (23), which can slide axially inside said cannulated wire guide (21) to be temporarily secured to the distal portion of a femur (4). Said guide wire (23) is used as a reference for removing a bone portion of the femur, thus obtaining a flattened surface on which a drillcentering jig is placed (27), through which seats for the fixation means (7) of the trochlear prosthesis (1) are formed. The equipment according to the invention allows for precise positioning of the trochlear prosthesis (1), particularly with respect to the trochlear sulcus of the femur. The invention further relates to a kit for fixing said prosthesis.

## Description

### Technical Field

The present invention relates to an equipment for the application of a trochlear prosthesis that has particular application in the veterinary field. The equipment of the present invention enables the trochlear prosthesis to be applied to a distal portion of the femur, at the knee joint, particularly at the femoral trochlea.

Specifically, the present invention relates to an equipment for the application of a trochlear prosthesis that is used for the treatment of certain joint diseases in animals, such as dogs. As a non-limiting example, the present invention relates to an equipment for the application of a trochlear prosthesis susceptible to be used particularly, but not exclusively, for the treatment of dogs suffering from patellar luxation with patellofemoral arthrosis.

### Background Art

As is known, patellar luxation in animals, particularly in dogs, is a very common orthopedic pathological condition, often due to a genetic predisposition. In the following description, special reference will be made to patellar luxation in the dog; however, it is to be understood that this pathology may occur in other animals as well.

This pathology leads to degeneration of the cartilage present on the patellofemoral joint surfaces as well as to the development of patellofemoral arthrosis, resulting in lameness, pain at the knee joint, and progressive reduction of the dog's motor capacity.

When the anatomy of the bone surfaces constituting the knee joint exceeds a certain degree of alteration, it is necessary to resort to surgery, aimed at restoring the correct form and function of the joint.

Typically, such surgery consists of replacing the altered bone portion with an artificial prosthesis, designed to simulate the functioning of one or more elements of the joint.

The altered bone portion may involve bone parts that make up the knee joint, such as the femoral trochlea.

Specifically, according to prior art, reconstruction of the articular surface of the femoral trochlea can be achieved by using a trochlear prosthesis that is implanted at the femur by surgery.

Typically, during surgery, the trochlear prosthesis is positioned on a distal part of the femur, at the knee joint, and is housed at the trochlear sulcus of the femur. This prosthesis is shaped in such a way as to define an artificial trochlear sulcus, which replaces the damaged femoral trochlea.

This makes it possible to relieve the dog's discomfort by reducing pain and lameness associated with osteoarthritis, to improve patellar stability so as to prevent dislocation, and, should the surgery require it, to correct the alignment of the extensor mechanism.

Various types of trochlear prostheses currently used in the veterinary field for reconstruction of the articular surface of the femoral trochlea are known.

As is known, in order to insert a trochlear prosthesis, a part of the distal portion of the femur must be properly shaped by removal of bone surfaces so that this shaped portion can accommodate the prosthesis.

In the veterinary field, this removal of bone tissue is not performed with special instruments, dedicated to the application of a trochlear prosthesis.

In addition, bone resection is, in general, performed 'by eye,' without the use of reference equipment. This leads to poor precision in bone resection and, consequently, poorly accurate positioning of the trochlear prosthesis.

The reduced precision of the surgery greatly increase the risk that the surgery will fail, often leading to the need for subsequent revision surgery.

In addition, the limitations described above lead to the need for a long postoperative course, during which, if there is no proper management by the veterinary doctor or by the owner, the risk of recurrence is greatly increased.

Thus, an object of the present invention is to overcome the drawbacks of prior art by providing medical personnel with special equipment to perform the implantation of a trochlear prosthesis to enable smooth and precise bone resection.

A further object of the present invention is to provide an equipment for the application of a trochlear prosthesis that will reduce the risk of any complications during surgery and the need for subsequent revisions by medical personnel.

Finally, it is an object of the present invention to provide an equipment for the application of a trochlear prosthesis that can be made easily and inexpensively so that, on the one hand, it can be produced in different sizes to take into account the femoral anatomy, which varies from patient to patient, and on the other hand, it can ensure a higher quality surgery without affecting the cost of the surgery itself.

These and other objects are achieved with the equipment for the application of a trochlear prosthesis as claimed in the appended claims.

### Summary of Invention

In the following description, the term 'distal' will denote the portion of an element or anatomical part that is farther from the center of a patient's body, the term 'proximal' will denote the portion of an element or anatomical part that is closer to the center of the patient's body, the term 'anterior' will denote the portion of an element or anatomical part facing the head of a patient, the term 'posterior' will denote the portion of an element or anatomical part facing away from the patient's head.

The present invention relates to an equipment for the application of a trochlear prosthesis suitable for veterinary use.

Said trochlear prosthesis is used in trochlear sulcus restoration procedures for restoring knee joint function in animals, such as in dogs. In particular, this trochlear prosthesis is used for the treatment of joint diseases in dogs, more particularly in the treatment of dogs suffering from patellar luxation with patellofemoral arthrosis.

Said trochlear prosthesis comprises a main body in which a trochlear sulcus is defined, fixation means allowing fixation of the trochlear prosthesis to a distal portion of a patient's femur and a trochlear extension apt to cooperate, in use, with a distal portion of the femur. The main body has a substantially concave, curved anterior face apt to accommodate the patient's patella, and a posterior supporting face, preferably flat, apt to be placed on a flattened surface at a distal portion of the femur.

The anterior face, facing the patella, is made concave so as to reproduce the anatomical shape of the femoral trochlear sulcus, allowing it to accommodate the patient's patella.

The trochlear extension is preferably made as a single piece with the main body.

This trochlear extension is located at the distal end of the main body and defines a prolongation of the concave anterior face of the main body. The trochlear extension is, in addition, connected to the posterior face of the main body, so as to define a posterior face of the trochlear extension that is not coplanar with the posterior face of the main body.

The trochlear extension in turn has a curved, substantially concave anterior face. The concave surface of the trochlear extension essentially reproduces the natural anatomical shape of the corresponding portion of the femur over which it is positioned at the time of implantation.

Preferably, the trochlear extension extends away from the posterior face of the main body and is shaped in such a way as to cooperate with said distal portion of the femur.

More preferably, the trochlear extension is associated with the main body in such a way as to define an obtuse angle with the posterior face of the main body.

Even more preferably, the trochlear extension has a curved, substantially C-shaped, cross-section.

Preferably, the fixation means include fixation pins located on the posterior face of the main body and/or the posterior face of the trochlear extension, said fixation pins being suitable to cooperate with appropriate fixation seats provided in the distal portion of the femur.

Said trochlear prosthesis can be made in different sizes and measures to fit the anatomical dimensions of the patient's joint.

The equipment according to the invention allows to prepare the femoral bone for implantation of said trochlear prosthesis.

In fact, as is known, in order to apply a trochlear prosthesis, it is necessary to properly shape a part of the distal portion of the femur by removal of bone surfaces so that this shaped portion can accommodate the prosthesis.

The equipment according to the invention comprises a guide assembly, configured to be placed at the distal portion of the femur, preferably at the distal condyles.

Said guide assembly comprises one or more guide elements that can be placed in contact with certain regions of the distal portion of the femur in order to enable precise and unambiguous positioning of said guide assembly on the distal portion of the femur.

Said guide elements may also include one or more alignment means in order to enable alignment of said guide assembly on the distal portion of the femur.

According to the invention, at least one of said guide elements comprises a handgrip and a cannulated wire guide associated whit said handgrip in such a way as to be able to slide axially inside it, said cannulated wire guide having an axial through-hole adapted to accommodate therein a guide wire which can slide axially inside said cannulated wire guide. Once the guide assembly is positioned on the femoral bone, by appropriately acting on said guide element comprising the handgrip and the cannulated wire guide, said guide wire is used to drill a hole inside the femoral bone, allowing said guide wire to be temporarily fixed to the bone.

This guide wire is used as a reference for positioning a cannulated milling tool, which is, in fact, equipped with an axial hole suitable to accommodate said guide wire.

Said milling tool, when placed in contact with the femur, allows a small portion of the bone surface to be removed in order to obtain a flattened surface on the distal portion of the femur, which will serve as a bearing surface for the posterior face of the main body of the prosthesis.

Said cannulated milling tool may be selected from milling tools currently known to orthopedic surgeons and already used in the veterinary field.

The equipment according to the invention also includes a drill-centering jig, which allows for the making of appropriate seats, on the flattened surface thus obtained, for the fixation of the trochlear prosthesis.

The drill-centering jig has a first end which is apt to come in contact with the distal portion of the femur and in which a first bearing surface is defined, apt to be placed on the flattened surface of the distal portion of the femur, and a second surface in which a trochlear protrusion is defined apt to be placed at the portion of the trochlear sulcus.

Said drill-centering jig includes a plurality of guide holes, wherein each of said guide holes is adapted to accommodate a drill bit. Said drill bits allow the femoral bone to be drilled in order to obtain corresponding seats suitable for cooperating with the fixation means of the trochlear prosthesis to allow implantation of the trochlear prosthesis into the femoral bone. Preferably, the trochlear protrusion if the drill-centering jig has the same shape as the trochlear extension of the trochlear prosthesis.

Thanks to the equipment according to the invention, the femoral bone is shaped in such a way as to obtain a bearing surface for the prosthesis and in such a way as to make appropriate seats for the insertion of the fixation pins.

The bearing surface is used to provide support only to the posterior face of the main body of the prosthesis, since the trochlear extension does not need any bone removal for its application (except for the creation of seats, if any, for the fixation pins provided on the posterior face of the trochlear extension).

Specifically, the trochlear extension can be accommodated within the trochlear sulcus without the need to remove part of the femoral trochlea and part of the femoral condyles; therefore, thus allowing preservation of the trochlear sulcus.

As a result, said trochlear prosthesis implanted using the equipment according to the invention is less invasive than currently employed prostheses. This has significant advantages in terms of postoperative recovery.

At the same time, the equipment according to the present invention enables precise positioning of the trochlear prosthesis, allowing alignment between the prosthesis and the trochlear sulcus of the patient's femur.

Overall, the reduced bone resection and precision of prosthesis placement allow for improved surgical quality, accelerating postoperative recovery and dramatically decreasing the need for subsequent surgical revision.

### Brief Description of Drawings

Further advantages and features of the present invention will become evident from the following detailed description of a preferred embodiment of the invention, given by way of non-limiting example with reference to the annexed drawings, in which:
- Fig.1: shows a perspective top view of a trochlear prosthesis implanted using the equipment according to the invention;
- Fig.2: shows a perspective bottom view of the trochlear prosthesis of Figure 1;
- Fig.3: shows a guide assembly for guiding the equipment according to the invention;
- Fig.4: shows the guide assembly of Figure 3 applied to the distal portion of a femur;
- Fig.5: shows a milling tool included in the equipment according to the invention;
- Fig.6: shows a drill-centering jig included in the equipment according to the invention;
- Fig.7: shows a drill-centering jig included in the equipment according to the invention;
- Fig.8: shows the drill-centering jig of Figure 6 and 7 applied to the distal portion of the femur;
- Fig.9: shows the trochlear prosthesis of Figure 1 and 2 applied to the distal portion of the femur.

### Description of Embodiments

In the following description, reference will be made to a equipment for the application of a trochlear prosthesis used in the trochlear sulcus replacement procedure to restore joint function in a dog with severe patellofemoral arthritis.

However, this embodiment should not be interpreted in a limiting sense, and the equipment according to the invention could be used to implant a trochlear prosthesis in order to replace the degenerated femoral trochlea as a result of other pathologies or trauma to the knee joint. In addition, such a equipment could be used on other animals besides the dog and could be used in combination with other commonly known equipment, such as that used on the patella and/or tibia.

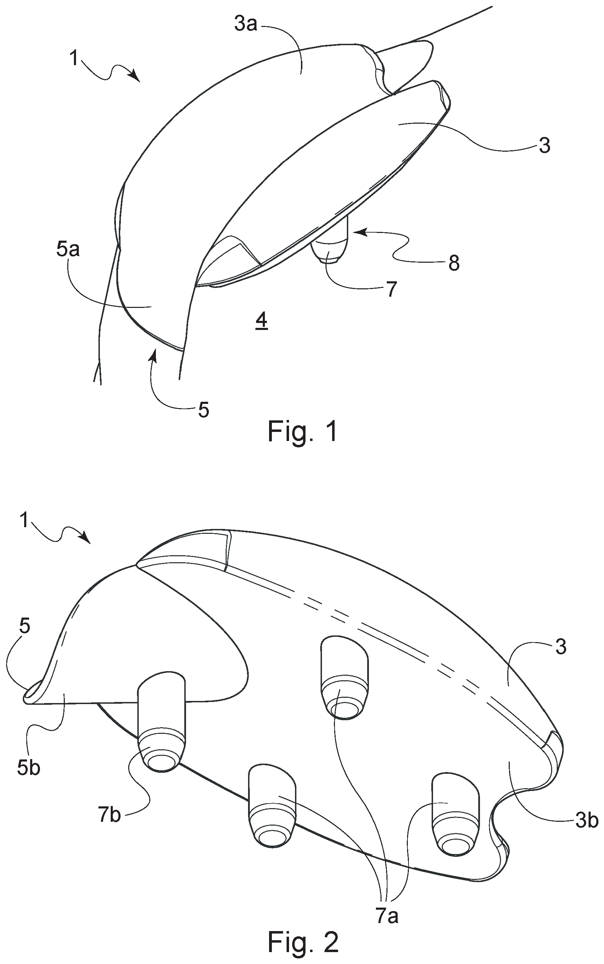

Figures 1 and 2 show a trochlear prosthesis, referred with reference numeral 1, which is implanted using the equipment according to the invention.

The trochlear prosthesis 1 comprises a main body 3 in which a trochlear sulcus is defined, fixation means 7 allowing fixation of the trochlear prosthesis 1 to a distal portion of a patient's femur 4, and a trochlear extension 5 apt to cooperate, in use, with the distal portion of the femur 4.

The main body 3 has a curved anterior face 3a, substantially concave in the illustrated embodiment, apt to accommodate the patient's patella. The main body 3 further has a posterior supporting face 3b, preferably flat, apt to be placed, when in use, on a flattened surface at a distal portion of the femur 4.

The anterior face 3a, facing, in use, the patient's patella, is made concave so as to reproduce the anatomical shape of the femoral trochlear sulcus, allowing it to accommodate the patient's patella.

The trochlear extension 5, in the illustrated embodiment, is made as a single piece with the main body 3 and is located at the distal end of the main body 3.

Due to this configuration, the trochlear extension 5 defines a prolongation of the anterior face 3a of the main body 3 and is connected to the posterior face 3b of the main body 3 so as to define a posterior face 5b of the trochlear extension 5 not coplanar with the posterior face 3b of the main body 3.

In the illustrated embodiment, the trochlear extension 5 in turn has a curved, substantially concave anterior face 5a.

The concave surface of the trochlear extension 5 reproduces the natural anatomical shape of the corresponding portion of the trochlear sulcus, over which it is placed at the time of implantation.

In the illustrated embodiment, the concave anterior face 3a of the main body 3 defines a single surface with the concave anterior face 5a of the trochlear extension.

In addition, the trochlear extension 5 extends away from the posterior face 3b of the main body 3, is shaped in such way as to cooperate with the distal portion of the femur 4 and is associated with the main body in such a way as to define an obtuse angle with the posterior face 3b of the main body 3.

In addition, in the illustrated embodiment, the trochlear extension 5 has a curved, substantially C-shaped, cross-section.

As can be seen from Figure 2, in the illustrated embodiment, the trochlear prosthesis 1 comprises three fixation pins 7a placed on the posterior face 3b of the main body 3 and a fixation pin 7a placed on the posterior face 5b of the trochlear extension 5 facing, in use, the patient's femur.

The fixation pins 7a, 7b allow fixation of the prosthesis at the distal portion of the femoral bone by cooperating with appropriate seats 8 provided in the femoral bone to accommodate said pins 7a, 7b.

In other embodiments, the fixation pins 7a, 7b and the corresponding seats 8 provided in the femoral bone can assume a configuration other than the illustrated one.

The trochlear prosthesis 1 can be produced in different sizes and measures so as to adapt to the anatomical sizes of the patient's joint.

In addition, the trochlear prosthesis 1 can be made of any material known to the person skilled in the art, such as titanium, and can also have different coating materials, which can be applied by different techniques, such as anodizing.

Said coating materials should be such that they provide high scratch resistance and a very low coefficient of friction between the surface of the trochlear prosthesis and the patella so as to allow smooth sliding of the patella, thus reducing the risk of inflammation.

Advantageously, the trochlear prosthesis 1 allows for a reduction in the removal of bone tissue required for the implantation of such prosthesis because the trochlear extension 5 can be accommodated within the trochlear sulcus without the need to remove part of the femoral trochlea and part of the femoral condyles.

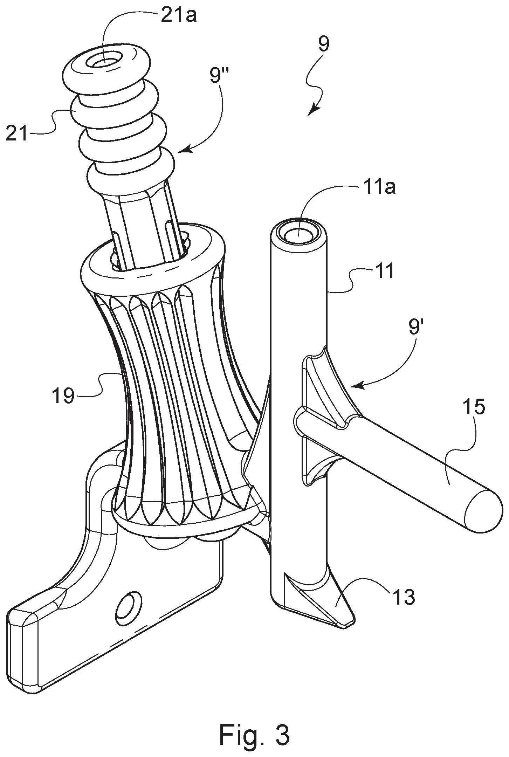

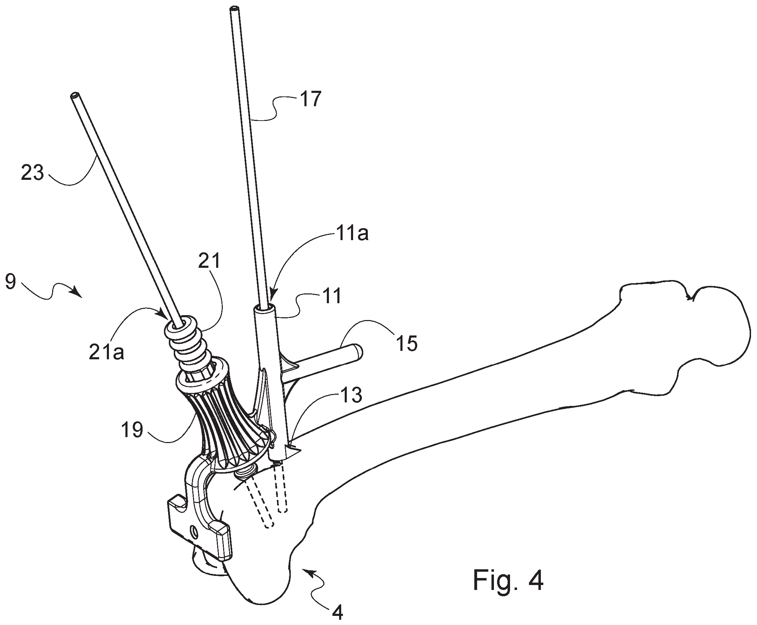

With reference to Figures 3 - 8, the equipment according to the invention is provided, which allows to prepare the femoral bone for implantation of said trochlear prosthesis 1.

Referring at first to Figures 3 - 4, the equipment according to the present invention comprises a guide assembly 9, configured to be placed at the distal portion of the femur 4, preferably at the distal condyles of the femur.

Said guide assembly 9 comprises one or more guide elements 9' and 9" which can be placed in contact with certain regions of the distal portion of the femur 4 in order to enable precise and unambiguous positioning of said guide assembly 9 on the distal portion of the femur 4. The guide assembly 9 comprises a first guide element 9' comprising in turn a turret 11 provided with a foot 13 configured to be placed on the anterior part of the femur, near the proximal end of the femoral condyles.

The first guide element comprises an alignment bar 15 enabling precise positioning of the guide assembly 9, so that said assembly is aligned to the femur. Said turret 11 is provided with an axial passageway 11a apt to accommodate therein a temporary fixation wire 17, which can slide axially inside said axial passageway 11a.

The guide assembly 9 further comprises a second guide element 9" comprising in turn a handgrip 19 and a cannulated wire guide 21. The cannulated wire guide 21 is associated with the handgrip 19 in such a way as to be able to slide axially inside said handgrip and has an axial through-hole 21a apt to accommodate therein a guide wire 23, which can slide axially inside said cannulated wire guide 21.

In order to proceed with bone resection, once the guide assembly 9 is placed on the femoral bone and the first drilling is performed inside the femoral bone by using the temporary fixation wire 17, the cannulated wire guide 21 is pushed until it comes in contact with the bone surface.

After that, the guide wire 23 with which a second hole is drilled inside the femoral bone is inserted inside the cannulated wire guide 21, temporarily securing said guide wire 23 to the bone.

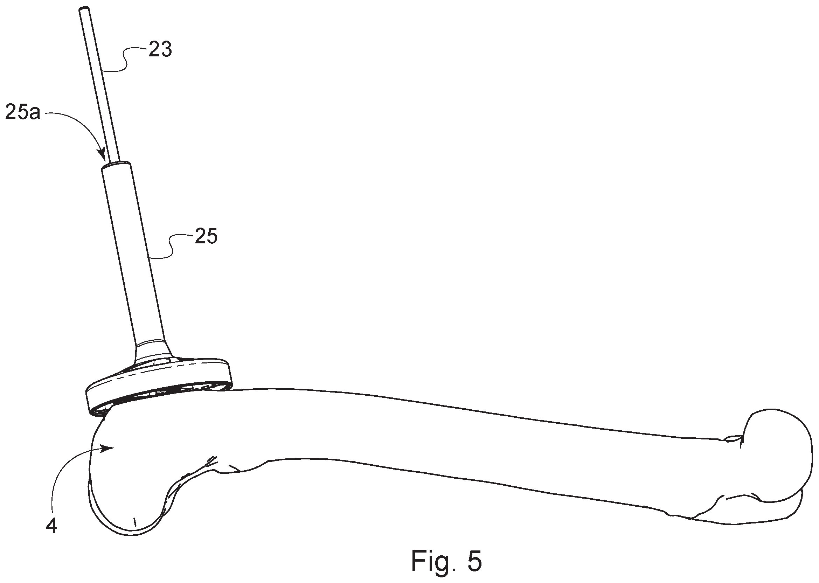

As can be seen from Figure 5, after removing the guide assembly 9 and the temporary fixation wire 17, the guide wire 23 is used as a reference for positioning a cannulated milling tool 25.

Said cannulated milling tool, in fact, is provided with an axial hole 25a apt to accommodate said guide wire 23.

Said cannulated milling tool 25, once placed in contact with the femur, allows removal of a small portion of the bone surface. This makes it possible to obtain a flattened surface on the distal portion of the femur 4, which will serve as a bearing surface for the posterior face 3b of the main body 3 of the prosthesis 1.

Said flattened surface obtained by milling will preferably be as flat and uniform as possible to subsequently facilitate correct fixation of the trochlear prosthesis 1.

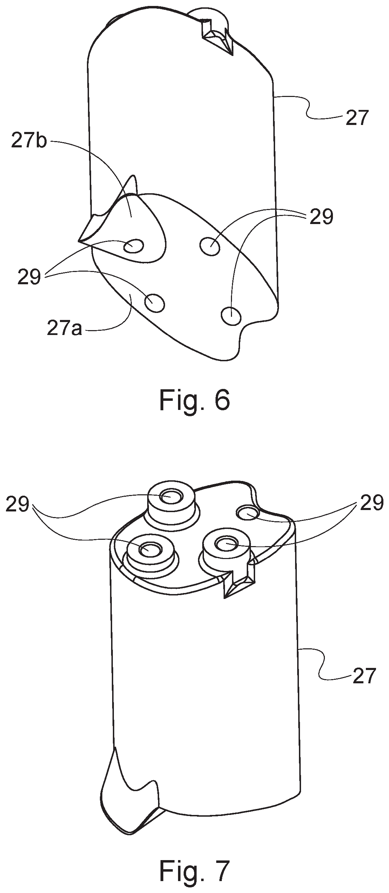

The equipment according to the invention further comprises a drill-centering jig 27, shown in Figure 6-8, which makes it possible to form, on the flattened surface thus obtained, appropriate seats 8 for fixing the trochlear prosthesis 1.

Said drill-centering jig 27 has a first end which is apt to come in contact with the distal portion of the femur 4 and in which a first bearing surface 27a is defined, apt to be placed on the flattened surface of the distal portion of the femur 4, and a second surface in which a trochlear protrusion 7b is defined.

Preferably, the trochlear protrusion 27a has the same shape as the trochlear extension 5.

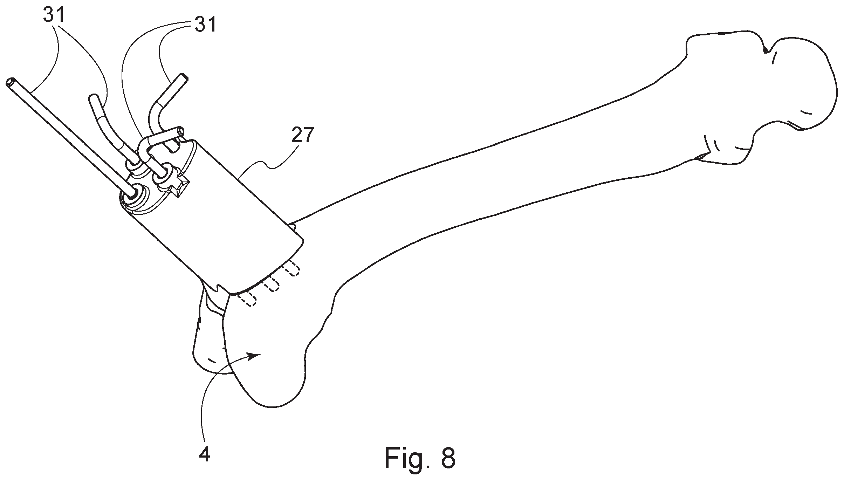

In the illustrated embodiment, the drill-centering jig 27 further has four guide holes 29. Each of said guide holes 29 is apt to accommodate a corresponding drilling bit 31, as shown in Figure 8. Said drilling bits 31 allow drilling of the femoral bone, in order to obtain corresponding seats 8 apt to accommodate the fixation pins 7 of the trochlear prosthesis 1. After making said seats 8 for the fixation pins 7, the drill-centering jig 27 is removed.

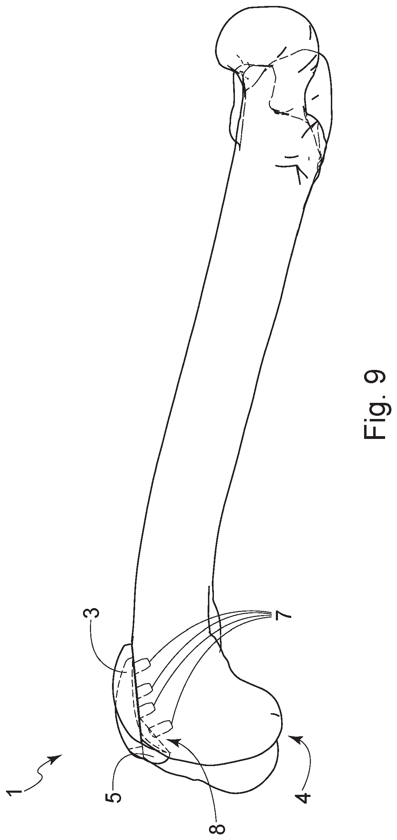

Finally, as shown in Figure 9, the trochlear prosthesis 1 is implanted on the femoral bone by placing the posterior face 3a of the main body 3 onto the flattened surface obtained by means of the milling tool 25 and by inserting the fixation pins 7 of the prosthesis 1 into the corresponding seats 8.

According to a preferred embodiment of the invention, the guide assembly 9 and the drill-centering jig 27 can be provided as a kit, preferably a disposable kit, together with the trochlear prosthesis 1 to be implanted in the femoral bone.

Even more preferably, the guide assembly 9 and the drill-centering jig 27 contained in the kit are made of plastics.

According to an even more preferred embodiment of the invention, not only the trochlear prostheses, but also said disposable kits can be manufactured in different sizes, so that, once the size of the trochlear prosthesis more suitable for the patient has been identified, the corresponding disposable kit can be associated with such prosthesis.

It will be apparent to the person skilled in the art that the embodiment described above in detail should in no way be taken as limiting, and that several variations and modifications are possible without thereby departing from the scope of the invention as defined by the appended claims.

## Claims

1. Equipment for applying a trochlear prosthesis (1), comprising:
- a guide assembly (9), configured to be placed at a distal portion of the femur (4) and comprising one or more guide elements (9', 9") which can be placed in contact with certain regions of the distal portion of the femur (4) in order to enable positioning of said guide assembly (9) on the distal portion of the femur (4), wherein at least one (9") of said guide elements comprises an axial through-hole (21a) apt to accommodate therein a guide wire (23), which can slide axially inside the guide element itself;
- a drill-centering jig (27) which has a first end which is apt to come in contact with the distal portion of the femur (4) and in which there are defined a first bearing surface (27a), apt to be placed on a flattened surface of the distal portion of the femur (4), and a second surface in which a trochlear protrusion (27b) is defined, said drill-centering jig (27) comprising a plurality of guide holes (29), each of said guide holes (29) being apt to accommodate a drilling bit (31);
**characterized in that** said at least one guide element (9") comprising the axial through-hole (21a) further comprises a handgrip (19) and a cannulated wire guide (21) associated therewith in such a way as to be able to slide axially inside said handgrip (19), said axial through-hole (21a) being provided in said cannulated wire guide (21).

2. Equipment according to claim 1, wherein at least one of said guide elements (9', 9") comprises a turret (11) provided with a foot (13) apt to be placed near a proximal end of the femoral condyles, wherein said turret (11) is provided with an axial passageway (11a) apt to accommodate therein a temporary fixation wire (17), which can slide axially inside said turret (11).

3. Equipment according to claim 2, wherein said at least one guide elements (9") comprising the axial through-hole (21a) is different from said at least one guide elements (9') comprising the turret (11) provided with the foot (13).

4. Equipment according to claim 1, 2 or 3, wherein at least one of said guide elements (9', 9") comprises alignment means for enabling alignment of said guide assembly (9) on the distal portion of the femur (4), and wherein said alignment means comprise an alignment bar (15).

5. Equipment according to claim 2, wherein said at least one guide element (9') comprising said turret (11) provided with said foot (13) further comprises alignment means for enabling alignment of said guide assembly (9) on the distal portion of the femur (4), and wherein said alignment means comprise an alignment bar (15).

6. Equipment according to any of the preceding claims, further comprising a cannulated milling tool (25) provided in turn with an axial hole (25a) apt to accommodate said guide wire (23).

7. Equipment according to any of the preceding claims, wherein said guide assembly (9) and said drill-centering jig (27) are made of plastics.

8. Kit comprising:
- an equipment (9, 27) according to any of claims 1 - 7; and
- a trochlear prosthesis (1) comprising:
- a main body (3) having a concave anterior face (3a) in which a trochlear sulcus is defined, said anterior face being apt to accommodate the patient's patella, and a posterior supporting face (3b), apt to be placed on a flattened surface of a distal portion of the patient's femur (4);
- fixation means (7) apt to allow fixation of said trochlear prosthesis (1) to the distal portion of the femur (4);
- a trochlear extension (5) which is arranged at an end of the main body (3) in such a way as to define a prolongation of said anterior face (3a), wherein said trochlear extension (5) is connected to said posterior face (3b) of the main body (3) in such a way as to define a posterior face (5b) of said trochlear extension (5) that is not coplanar with said posterior face (3b) of the main body (3).

9. Kit according to claim 8, wherein the trochlear protrusion (27b) of the drill-centering jig (27) has the same shape as the trochlear extension (5) of the trochlear prosthesis (1).
